# EUROPÄISCHE PATENTANMELDUNG

(11) **EP 1 457 569 A1**
(43) Veröffentlichungstag der Anmeldung: **15.09.2004**
(21) Anmeldenummer: 04005193.0
(22) Anmeldetag: 04.03.2004
(51) Int. Cl.: C12P 19/40

(54) **Verfahren zur fermentativen Herstellung von S-Adenosylmethionin**

(30) Priorität: 06.03.2003 DE 10309856
(71) Anmelder: Consortium für elektrochemische Industrie GmbH, 81379 München (DE)
(72) Erfinder: Leonhartsberger, Susanne, Dr., 80634 München (DE); Maier, Thomas, Dr., 85221 Dachau (DE)
(74) Vertreter: Potten, Holger

(57) **Zusammenfassung**

Verfahren zur fermentativen Produktion von S-Adenosylmethionin (SAM), dadurch gekennzeichnet, daß ein Bakterienstamm, der aus einem Ausgangsstamm erhältlich ist, und eine im Vergleich zu dem Ausgangsstamm verstärkte Aktivität der SAM-Synthetase aufweist, in einem Kulturmedium kultiviert wird, wobei der Bakterienstamm SAM in das Kulturmedium ausscheidet und das SAM vom Kulturmedium abgetrennt wird.

SEQUENCE LISTING

<110> Consortium fuer elektrochemische Industrie GmbH

<120> Prozess zur fermentativen Herstellung von
      S-Adenosylmethionin

<130> Co10302

<140>
<141>

<160> 11

<170> PatentIn Ver. 2.0

<210> 1
<211> 384
<212> PRT
<213> Escherichia coli

<400> 1
Met Ala Lys His Leu Phe Thr Ser Glu Ser Val Ser Glu Gly His Pro
  1               5                  10                  15

Asp Lys Ile Ala Asp Gln Ile Ser Asp Ala Val Leu Asp Ala Ile Leu
             20                  25                  30

Glu Gln Asp Pro Lys Ala Arg Val Ala Cys Glu Thr Tyr Val Lys Thr
         35                  40                  45

Gly Met Val Leu Val Gly Gly Glu Ile Thr Thr Ser Ala Trp Val Asp
    50                  55                  60
Ile Glu Glu Ile Thr Arg Asn Thr Val Arg Glu Ile Gly Tyr Val His
65                  70                  75                  80
Ser Asp Met Gly Phe Asp Ala Asn Ser Cys Ala Val Leu Ser Ala Ile
                85                  90                  95
Gly Lys Gln Ser Pro Asp Ile Asn Gln Gly Val Asp Arg Ala Asp Pro
            100                 105                 110
Leu Glu Gln Gly Ala Gly Asp Gln Gly Leu Met Phe Gly Tyr Ala Thr
        115                 120                 125
Asn Glu Thr Asp Val Leu Met Pro Ala Pro Ile Thr Tyr Ala His Arg
    130                 135                 140
Leu Val Gln Arg Gln Ala Glu Val Arg Lys Asn Gly Thr Leu Pro Trp
145                 150                 155                 160
Leu Arg Pro Asp Ala Lys Ser Gln Val Thr Phe Gln Tyr Asp Asp Gly
                165                 170                 175
Lys Ile Val Gly Ile Asp Ala Val Val Leu Ser Thr Gln His Ser Glu
            180                 185                 190
Glu Ile Asp Gln Lys Ser Leu Gln Glu Ala Val Met Glu Glu Ile Ile
        195                 200                 205

Lys Pro Ile Leu Pro Ala Glu Trp Leu Thr Ser Ala Thr Lys Phe Phe
    210                 215                 220

Ile Asn Pro Thr Gly Arg Phe Val Ile Gly Gly Pro Met Gly Asp Cys
225                 230                 235                 240

Gly Leu Thr Gly Arg Lys Ile Ile Val Asp Thr Tyr Gly Gly Met Ala
                245                 250                 255

Arg His Gly Gly Gly Ala Phe Ser Gly Lys Asp Pro Ser Lys Val Asp
            260                 265                 270

Arg Ser Ala Ala Tyr Ala Ala Arg Tyr Val Ala Lys Asn Ile Val Ala
        275                 280                 285

Ala Gly Leu Ala Asp Arg Cys Glu Ile Gln Val Ser Tyr Ala Ile Gly
    290                 295                 300

Val Ala Glu Pro Thr Ser Ile Met Val Glu Thr Phe Gly Thr Glu Lys
305                 310                 315                 320

Val Pro Ser Glu Gln Leu Thr Leu Leu Val Arg Glu Phe Phe Asp Leu
                325                 330                 335

Arg Pro Tyr Gly Leu Ile Gln Met Leu Asp Leu Leu His Pro Ile Tyr
            340                 345                 350

Lys Glu Thr Ala Ala Tyr Gly His Phe Gly Arg Glu His Phe Pro Trp
        355                 360                 365

Glu Lys Thr Asp Lys Ala Gln Leu Leu Arg Asp Ala Ala Gly Leu Lys
    370                 375                 380

<210> 2
<211> 1155
<212> DNA
<213> Escherichia coli

<220>
<221> gene
<222> (1)..(1152)
<223> metK

<300>
<301> Blattner, F. R.
<302> The complete genome sequence of Escherichia coli K-12.
<303> Science
<304> 277
<306> 1453-1474
<307> 1997

<400> 2
atggcaaaac acctttttac gtccgagtcc gtctctgaag ggcatcctga caaaattgct 60

gaccaaattt ctgatgccgt tttagacgcg atcctcgaac aggatccgaa agcacgcgtt 120

gcttgcgaaa cctacgtaaa aaccggcatg gttttagttg gcggcgaaat caccaccagc 180

gcctgggtag acatcgaaga gatcacccgt aacaccgttc gcgaaattgg ctatgtgcat 240

tccgacatgg gctttgacgc taactcctgt gcggttctga gcgctatcgg caaacagtct 300

cctgacatca accagggcgt tgaccgtgcc gatccgctgg aacagggcgc gggtgaccag 360

ggtctgatgt ttggctacgc aactaatgaa accgacgtgc tgatgccagc acctatcacc 420

tatgcacacc gtctggtaca gcgtcaggct gaagtgcgta aaaacggcac tctgccgtgg 480

ctgcgcccgg acgcgaaaag ccaggtgact tttcagtatg acgacggcaa aatcgttggt 540

atcgatgctg tcgtgctttc cactcagcac tctgaagaga tcgaccagaa atcgctgcaa 600

gaagcggtaa tggaagagat catcaagcca attctgcccg ctgaatggct gacttctgcc 660

accaaattct tcatcaaccc gaccggtcgt ttcgttatcg gtggcccaat gggtgactgc 720

ggtctgactg gtcgtaaaat tatcgttgat acctacggcg gcatggcgcg tcacggtggc 780

ggtgcattct ctggtaaaga tccatcaaaa gtggaccgtt ccgcagccta cgcagcacgt 840

tatgtcgcga aaaacatcgt tgctgctggc ctggccgatc gttgtgaaat tcaggtttcc 900

tacgcaatcg gcgtggctga accgacctcc atcatggtag aaactttcgg tactgagaaa 960

gtgccttctg aacaactgac cctgctggta cgtgagttct tcgacctgcg cccatacggt 1020

ctgattcaga tgctggatct gctgcacccg atctacaaag aaaccgcagc atacggtcac 1080

tttggtcgtg aacatttccc gtgggaaaaa accgacaaag cgcagctgct gcgcgatgct 1140

gccggtctga agtaa 1155

<210> 3

<211> 29

<212> DNA

<213> Artificial Sequence

<220>

<223> Description of Artificial Sequence: Oligonukleotid

metK2

<400> 3

ccttaattaa tgtctgttgt ggttggtgt 29

<210> 4

<211> 29

<212> DNA

<213> Artificial Sequence

<220>

<223> Description of Artificial Sequence: Oligonukleotid

metK4

<400> 4

ggaattctct ttaggaggta ttaaatatg 29

<210> 5

<211> 37

<212> DNA

<213> Artificial Sequence

<220>

<223> Description of Artificial Sequence: Oligonukleotid

RLSS1

<400> 5

ctagcaggag gaattcacca tgggacctgt ggatggc 37

<210> 6

<211> 40

<212> DNA

<213> Artificial Sequence

<220>

<223> Description of Artificial Sequence: Oligonukleotid

RLSS2

<400> 6

gggtaccccg ctaaaacaca agcttcttgg ggacctccca 40

<210> 7

<211> 33

<212> DNA

<213> Artificial Sequence

<220>

<223> Description of Artificial Sequence: Oligonukleotid
      GAPDHfw

<400> 7
gtcgacgcgt gaggcgagtc agtcgcgtaa tgc                                33

<210> 8
<211> 43
<212> DNA
<213> Artificial Sequence

<220>
<223> Description of Artificial Sequence: Oligonukleotid
      GAPDHrevII

<400> 8
gaccttaatt aagatctcat atattccacc agctatttgt tag                     43

<210> 9
<211> 46
<212> DNA
<213> Artificial Sequence

<220>

<223> Description of Artificial Sequence: Multiple
      Cloning Site

<400> 9
gaagatctag gaggcctagc atatgtgaat tcccgggctg cagctg                46

<210> 10
<211> 1185
<212> DNA
<213> Rattus norvegicus

<220>
<221> CDS
<222> (1)..(1185)
<223> RLSS-Gen

<400> 10
atg gga cct gtg gat ggc ttg tgt gac cat tct cta agt gaa gag gga    48
Met Gly Pro Val Asp Gly Leu Cys Asp His Ser Leu Ser Glu Glu Gly
  1               5                  10                  15

gcc ttc atg ttc aca tct gaa tcg gta gga gaa ggg cat cca gat aag    96
Ala Phe Met Phe Thr Ser Glu Ser Val Gly Glu Gly His Pro Asp Lys
             20                  25                  30

atc tgt gac cag att agt gat gca gtg ctg gat gcc cat ctc aag caa    144
Ile Cys Asp Gln Ile Ser Asp Ala Val Leu Asp Ala His Leu Lys Gln
         35                  40                  45

gac ccc aat gcc aag gtg gcc tgt gag aca gtg tgc aag aca ggg atg 192
Asp Pro Asn Ala Lys Val Ala Cys Glu Thr Val Cys Lys Thr Gly Met
    50                  55                  60

gtg ctc ctg tgt gga gag atc acc tca atg gcc atg att gac tac cag 240
Val Leu Leu Cys Gly Glu Ile Thr Ser Met Ala Met Ile Asp Tyr Gln
65                  70                  75                  80

cgg gtg gtg aga gac acc att aag cac att ggc tac gat gac tct gcc 288
Arg Val Val Arg Asp Thr Ile Lys His Ile Gly Tyr Asp Asp Ser Ala
                85                  90                  95

aag ggc ttc gac ttc aag acc tgc aat gtg ctc gtg gct ctg gag caa 336
Lys Gly Phe Asp Phe Lys Thr Cys Asn Val Leu Val Ala Leu Glu Gln
            100                 105                 110

cag tcc cca gac att gcc caa tgt gtc cat cta gac aga aat gag gag 384
Gln Ser Pro Asp Ile Ala Gln Cys Val His Leu Asp Arg Asn Glu Glu
        115                 120                 125

gac gtt ggt gca gga gat cag ggt ctg atg ttc ggc tat gcc act gac 432
Asp Val Gly Ala Gly Asp Gln Gly Leu Met Phe Gly Tyr Ala Thr Asp
    130                 135                 140

gag aca gag gag tgc atg ccg ctc acc att gtt ctt gct cac aaa ctc 480
Glu Thr Glu Glu Cys Met Pro Leu Thr Ile Val Leu Ala His Lys Leu
145                 150                 155                 160

aac acc cgg atg gca gat ctg agg cgc tct ggt gtc ctt ccc tgg ctg 528
Asn Thr Arg Met Ala Asp Leu Arg Arg Ser Gly Val Leu Pro Trp Leu

                165                 170                 175

aga cct gat tct aag act cag gta aca gtt cag tac gtg cag gat aat 576

Arg Pro Asp Ser Lys Thr Gln Val Thr Val Gln Tyr Val Gln Asp Asn

            180                 185                 190

ggt gct gtc atc cct gtt cgc gtc cac acc atc gtc atc tct gtg caa 624

Gly Ala Val Ile Pro Val Arg Val His Thr Ile Val Ile Ser Val Gln

        195                 200                 205

cac aac gaa gac ata aca ctg gag gcc atg cga gag gcc ctg aag gag 672

His Asn Glu Asp Ile Thr Leu Glu Ala Met Arg Glu Ala Leu Lys Glu

    210                 215                 220

cag gtg atc aaa gct gtg gtg cca gcc aag tac ctg gat gaa gac acc 720

Gln Val Ile Lys Ala Val Val Pro Ala Lys Tyr Leu Asp Glu Asp Thr

225                 230                 235                 240

atc tac cac ctg cag cca agt ggg cgg ttt gtc atc gga ggt ccc cag 768

Ile Tyr His Leu Gln Pro Ser Gly Arg Phe Val Ile Gly Gly Pro Gln

                245                 250                 255

ggg gat gca ggt gtc aca ggc cgc aag att att gtg gac aca tac gga 816

Gly Asp Ala Gly Val Thr Gly Arg Lys Ile Ile Val Asp Thr Tyr Gly

            260                 265                 270

ggc tgg ggt gcc cat ggt ggt ggt gcc ttc tct gga aag gac tac acc 864

Gly Trp Gly Ala His Gly Gly Gly Ala Phe Ser Gly Lys Asp Tyr Thr

        275                 280                 285

aag gtg gac cgc tca gca gct tat gcc gcc cgc tgg gtg gcc aag tct 912
Lys Val Asp Arg Ser Ala Ala Tyr Ala Ala Arg Trp Val Ala Lys Ser
    290                 295                 300

ctg gtg aag gct ggg ctc tgc cgg aga gtc ctt gtt cag gtg tcc tat 960
Leu Val Lys Ala Gly Leu Cys Arg Arg Val Leu Val Gln Val Ser Tyr
305                 310                 315                 320

gcc att ggt gtg gca gaa cct ctg tcc att tcc att ttc acc tac gga 1008
Ala Ile Gly Val Ala Glu Pro Leu Ser Ile Ser Ile Phe Thr Tyr Gly
                325                 330                 335

act tcc aag aag acc gag cga gag cta cta gag gtt gtg aac aag aac 1056
Thr Ser Lys Lys Thr Glu Arg Glu Leu Leu Glu Val Val Asn Lys Asn
            340                 345                 350

ttt gac ctc cgg ccg ggt gtt att gtc agg gac ttg gat ctg aag aag 1104
Phe Asp Leu Arg Pro Gly Val Ile Val Arg Asp Leu Asp Leu Lys Lys
        355                 360                 365

ccc atc tac cag aag act gca tgc tat ggt cat ttc gga aga agc gag 1152
Pro Ile Tyr Gln Lys Thr Ala Cys Tyr Gly His Phe Gly Arg Ser Glu
    370                 375                 380

ttt ccc tgg gag gtc ccc aag aag ctt gtg ttt 1185
Phe Pro Trp Glu Val Pro Lys Lys Leu Val Phe
385                 390                 395

<210> 11

<211> 395

<212> PRT

<213> Rattus norvegicus

<400> 11

Met Gly Pro Val Asp Gly Leu Cys Asp His Ser Leu Ser Glu Glu Gly
1               5                   10                  15

Ala Phe Met Phe Thr Ser Glu Ser Val Gly Glu Gly His Pro Asp Lys
            20                  25                  30

Ile Cys Asp Gln Ile Ser Asp Ala Val Leu Asp Ala His Leu Lys Gln
        35                  40                  45

Asp Pro Asn Ala Lys Val Ala Cys Glu Thr Val Cys Lys Thr Gly Met
    50                  55                  60

Val Leu Leu Cys Gly Glu Ile Thr Ser Met Ala Met Ile Asp Tyr Gln
65                  70                  75                  80

Arg Val Val Arg Asp Thr Ile Lys His Ile Gly Tyr Asp Asp Ser Ala
                85                  90                  95

Lys Gly Phe Asp Phe Lys Thr Cys Asn Val Leu Val Ala Leu Glu Gln
            100                 105                 110

Gln Ser Pro Asp Ile Ala Gln Cys Val His Leu Asp Arg Asn Glu Glu
        115                 120                 125

Asp Val Gly Ala Gly Asp Gln Gly Leu Met Phe Gly Tyr Ala Thr Asp

130                 135                 140

Glu Thr Glu Glu Cys Met Pro Leu Thr Ile Val Leu Ala His Lys Leu
145                 150                 155                 160

Asn Thr Arg Met Ala Asp Leu Arg Arg Ser Gly Val Leu Pro Trp Leu
                165                 170                 175

Arg Pro Asp Ser Lys Thr Gln Val Thr Val Gln Tyr Val Gln Asp Asn
            180                 185                 190

Gly Ala Val Ile Pro Val Arg Val His Thr Ile Val Ile Ser Val Gln
        195                 200                 205

His Asn Glu Asp Ile Thr Leu Glu Ala Met Arg Glu Ala Leu Lys Glu
    210                 215                 220

Gln Val Ile Lys Ala Val Val Pro Ala Lys Tyr Leu Asp Glu Asp Thr
225                 230                 235                 240

Ile Tyr His Leu Gln Pro Ser Gly Arg Phe Val Ile Gly Gly Pro Gln
                245                 250                 255

Gly Asp Ala Gly Val Thr Gly Arg Lys Ile Ile Val Asp Thr Tyr Gly
            260                 265                 270

Gly Trp Gly Ala His Gly Gly Gly Ala Phe Ser Gly Lys Asp Tyr Thr
        275                 280                 285

Lys Val Asp Arg Ser Ala Ala Tyr Ala Ala Arg Trp Val Ala Lys Ser

    290                 295                 300

Leu Val Lys Ala Gly Leu Cys Arg Arg Val Leu Val Gln Val Ser Tyr

305                 310                 315                 320

Ala Ile Gly Val Ala Glu Pro Leu Ser Ile Ser Ile Phe Thr Tyr Gly

                325                 330                 335

Thr Ser Lys Lys Thr Glu Arg Glu Leu Leu Glu Val Val Asn Lys Asn

            340                 345                 350

Phe Asp Leu Arg Pro Gly Val Ile Val Arg Asp Leu Asp Leu Lys Lys

        355                 360                 365

Pro Ile Tyr Gln Lys Thr Ala Cys Tyr Gly His Phe Gly Arg Ser Glu

    370                 375                 380

Phe Pro Trp Glu Val Pro Lys Lys Leu Val Phe

385                 390                 395

## Beschreibung

Die Erfindung betrifft ein Verfahren zur fermentativen Herstellung von S-Adenosylmethionin unter Verwendung eines Bakterienstammes, der S-Adenosylmethionin-Synthetase überproduziert.

S-Adenosylmethionin (SAM) ist der wichtigste Methylgruppendonor im Stoffwechsel und findet im Pharmabereich Verwendung bei der Behandlung von Depressionen, Erkrankungen der Leber und Arthritis. Beschriebene Verfahren zur SAM-Herstellung umfassen die Anzucht von Hefen (Schlenk F. und DePalma R.E., J. Biol. Chem. 1037-1050 (1957), Shiozaki S. et.al., Agric. Biol. Chem. 53, 3269-3274 (1989)) in Gegenwart der Vorstufe L-Methionin und die chromatographische Aufreinigung des gebildeten SAM nach Extraktion aus dem Zelllysat (US4562149). Ein Nachteil dieses Verfahrens ist vor allem die aufwändige Aufreinigung des gebildeten SAM, da die Zellen zuerst aufgebrochen und SAM von allen anderen Zellbestandteilen wie Aminosäuren, Zuckern, Lipiden, Nukleotiden, Proteinen, Cofaktoren und anderen hoch- und niedermolekularen Verbindungen abgetrennt werden muß. Aus diesem Grund würde die Entwicklung eines Verfahrens zur fermentativen Produktion von SAM gegenüber bestehenden Verfahren einen deutlichen Vorteil aufweisen, wenn eine selektive Sekretion des gebildeten SAM in den Kulturüberstand und damit eine Vereinfachung des Aufreinigungsverfahrens möglich wäre. Im Kulturüberstand befinden sich nur wenige Substanzen, weshalb eine Sekretion von SAM schon einen ersten Aufreinigungsschritt darstellen und die weitere Aufreinigung deutlich erleichtern würde.

In GB1436509 ist ein Verfahren zur extrazellulären Produktion von SAM durch Hefen, wie zum Beispiel Candida tropicalis, beschrieben. Ein Nachteil dieses Verfahrens liegt darin begründet, daß die verwendeten Produktionsstämme ungewöhnliche Pilze sind, die nicht den GRAS-Status (generally recognized as safe) aufweisen, sondern zum Teil sogar als pathogene Organismen einzustufen sind. Außerdem sind diese Organismen genetischen Methoden schwer zugänglich und ihr Stoffwechsel ist weitgehend unbekannt. Damit fehlen zwei wesentliche Voraussetzungen für die Verbesserung durch metabolic engineering. Bakterien dagegen sind genetisch leicht zugänglich, der Stoffwechsel ist von vielen Arten gut erforscht und es gibt viele apathogene Arten, die den GRAS-Status aufweisen. Ein Verfahren, bei dem SAM durch Bakterien produziert wird, wäre daher sehr wünschenswert. Eine extrazelluläre Produktion von SAM in Bakterien ist bisher jedoch nicht bekannt.

Die Synthese von S-Adenosylmethionin wurde besonders intensiv im Bakterium Escherichia coli (E. coli) untersucht (Greene, R.C., Biosynthesis of Methionine in: Neidhardt F.C., Escherichia coli and Salmonella typhimurium, Cellular and molecular biology, Second Edition, ASM Press, Washington DC (1996), Seiten 542-560 und darin enthaltenen Referenzen). Die Synthese von SAM erfolgt im Anschluß an die aufwändige und hochregulierte Synthese von L-Methionin in einem Schritt aus L-Methionin und ATP. Dabei werden alle drei Phosphatgruppen aus ATP freigesetzt unter Entstehung von anorganischem Phosphat und Pyrophosphat. Diese Reaktion wird durch das Enzym S-Adenosylmethionin-Synthetase (EC 2.5.1.6, Methionin-Adenosyltransferase, SAM-Synthetase) katalysiert, das in E. coli durch das Gen *metK* codiert wird. Dieses Enzym wurde biochemisch und genetisch eingehend charakterisiert und zeigt eine sehr strenge Endprodukthemmung (Feedback-Regulation). Das heißt, die Aktivität des Enzyms ist in Anwesenheit eines Überschusses an SAM stark gehemmt (Markham et al., J. Biol. Chem., 9082-9092 (1980)). Diese Feedback-Regulation verhindert eine energieverbrauchende unnötige Synthese von SAM und einen zu hohen, für die Zelle möglicherweise schädlichen SAM-Spiegel in der Zelle, steht aber auch einer fermentativen Überproduktion von SAM im Weg. SAM-Synthetasen anderer Organismen (Saccharomyces cerevisiae, Methanococcus janaschii, Ratte) wurden ebenfalls untersucht, sie zeigen ebenfalls eine Hemmbarkeit durch SAM, die jedoch nicht so ausgeprägt wie bei der SAM-Synthetase von E. coli ist (Park et al., Bioorgan. Med. Chem., 2179-2185 (1996); Lu and Markham, J. Biol. Chem., 16624-16631 (2002); Oden und Clarke, Biochemistry, 2978-2986 (1983).

Im Gegensatz zu anderen Organismen (z.B. Hefe) existiert in Bakterien kein Transportsystem für SAM und Bakterien können diese Substanz daher nicht aus dem Medium aufnehmen, weshalb die SAM-Synthetase ein essentielles Enzym ist. Die SAM-Synthetase von E. coli konnte überproduziert werden, was zu einer Zunahme der Enzym-Menge in der Zelle führte (Markham et al., J. Biol. Chem., 9082-9092 (1980)). Es ist jedoch nicht bekannt, daß eine Überproduktion auch zu einer Zunahme der SAM-Menge in der Zelle führt. Dies ist auch nicht zu erwarten, da eine Anhäufung von SAM in der Zelle durch oben erwähnte Feedback-Regulation der SAM-Synthetase verhindert wird. Die intrazelluläre Produktion von SAM ist also durch die Regulation der Aktivität der SAM-Synthetase limitiert.

Dagegen konnte durch Überproduktion der SAM-Synthetase aus der Rattenleber der intrazelluläre SAM-Spiegel in E. coli deutlich erhöht werden (Alvarez et al., Biochem. J., 557-561 (1994); EP0647712A1). Dies ist möglich, weil diese SAM-Synthetase keiner strengen Feedback-Regulation unterliegt wie das homologe Enzym aus E. coli und die Regulation in dem Bakterium so umgangen wird. Jedoch wurde auch hier keine extrazelluläre Akkumulation von SAM beobachtet.

Aufgabe der vorliegenden Erfindung ist es, ein Verfahren zur fermentativen Produktion von SAM mittels Bakterien zur Verfügung zu stellen, welches die Aufreinigung von SAM deutlich vereinfacht.

Diese Aufgabe wird gelöst durch ein Verfahren, welches dadurch gekennzeichnet ist, dass ein Bakterienstamm, der aus einem Ausgangsstamm mit einer SAM-Synthetase erhältlich ist und eine im Vergleich zu dem Ausgangsstamm verstärkte Aktivität der SAM-Synthetase aufweist, in einem Kulturmedium kultiviert wird, wobei der Bakterienstamm SAM in das Kulturmedium ausscheidet und das SAM vom Kulturmedium abgetrennt wird.

Angesichts der Tatsache, daß die SAM-Synthetase in Bakterien, vor allem in E. coli, wie oben beschrieben einer strengen Feedback-Regulation unterliegt, ist es überraschend, daß bei Steigerung der Aktivität eine Zunahme der SAM-Produktion zu beobachten ist. Insbesondere ist es völlig unerwartet, daß das überproduzierte SAM in den Kulturüberstand ausgeschieden wird. Wie oben beschrieben existieren keine Beispiele, daß fermentativ hergestelltes SAM von Bakterien in den Kulturüberstand abgegeben wird. Insbesondere ist in Bakterien kein TransportSystem für SAM bekannt und SAM kann auch nicht aus dem Medium aufgenommen werden. Eine passive Diffusion nach außen ist bei einem derart großen und außerdem geladenen Molekül wie SAM extrem unwahrscheinlich. Daher ist die extrazelluläre Anreicherung von SAM für den Fachmann völlig überraschend.

Die Vorteile des erfindungsgemäßen Verfahrens ergeben sich aus der gesteigerten SAM-Produktion und der erleichterten Aufarbeitung aus dem Kulturüberstand. Dieses Verfahren ermöglicht eine gesteigerte SAM-Produktion auch durch SAM-Synthetasen, die normalerweise einer strengen Produkthemmung unterworfen sind, welche eine intrazelluläre Anhäufung von SAM verhindert, dadurch daß das produzierte SAM in den Kulturüberstand sekretiert wird und damit die SAM-Synthetase nicht mehr hemmt.

Darüberhinaus wurde überraschend festgestellt, dass.im Gegensatz zum Stand der Technik in dem erfindungsgemäßen Verfahren anstelle von L-Methionin als Vorläufer auch D,L-Methionin verwendet werden kann. Letzteres ist erheblich kostengünstiger und erlaubt somit eine drastische Ersparnis bei den Herstellkosten.

Die Erfindung betrifft somit auch ein Verfahren, welches dadurch gekennzeichnet ist, dass ein Bakterienstamm, der aus einem Ausgangsstamm mit einer SAM-Synthetase erhältlich ist und eine im Vergleich zu dem Ausgangsstamm verstärkte Aktivität der SAM-Synthetase aufweist, in einem Kulturmedium kultiviert wird, welches D,L-Methionin enthält, wobei der Bakterienstamm SAM in das Kulturmedium ausscheidet und das SAM vom Kulturmedium abgetrennt wird.

Vorzugsweise wird im erfindungsgemäßen Verfahren als SAM-Synthetase ein Protein umfassend die Sequenz SEQ ID NO: 1 oder funktionelle Varianten mit einer Sequenzähnlichkeit zu SEQ ID NO: 1 größer 40 % eingesetzt.

Vorzugsweise ist die Sequenzähnlichkeit zu SEQ ID NO: 1 größer 60 %, besonders bevorzugt ist die Sequenzähnlichkeit zu SEQ ID NO: 1 größer 80 %.

In der vorliegenden Erfindung beziehen sich alle erwähnten Homologiewerte auf Ergebnisse, die mit dem Algorithmus BESTFIT (GCG Wisconsin Package, Genetics Computer Group (GCG) Madison, Wisconsin) erhalten werden. Die Erfindung betrifft auch die vorgenannten SAM-Synthetasen.

Vorzugsweise wird im erfindungsgemäßen Verfahren ein Gen für eine der vorgenannten SAM-Synthetasen, im Folgenden auch als *metK*-Gen bezeichnet, verwendet. Es handelt sich dabei um ein Gen mit der Sequenz SEQ ID NO: 2 oder um eine funktionelle Variante dieses Gens.

Unter einer funktionellen Variante ist im Sinne der vorliegenden Erfindung eine DNS-Sequenz zu verstehen, die sich durch Deletion, Insertion oder Substitution von Nukleotiden aus der in SEQ ID NO: 2 dargestellten Sequenz ableitet, wobei die enzymatische Aktivität der durch das Gen codierten SAM-Synthetase erhalten bleibt.

Unter einer verstärkten Aktivität ist im Sinne der vorliegenden Erfindung vorzugsweise zu verstehen, dass die Aktivität der SAM-Synthetase in einem erfindungsgemäß verwendeten Bakterienstamm im Vergleich zum jeweiligen Ausgangsstamm mindestens um den Faktor 2, bevorzugt mindestens um den Faktor 5 gesteigert ist.

Bakterienstämme, die im erfindungsgemäßen Verfahren eingesetzt werden und eine gegenüber einem Ausgangsstamm erhöhte SAM-Synthetase Aktivität aufweisen, können mit Standardtechniken der Molekularbiologie aus einem Ausgangsstamm, in der Regel einem Wildtyp-Stamm, erzeugt werden.

In einer Vielzahl von Ausgangsstämmen konnten SAM-Synthetase-Gene identifiziert werden. Bakterienstämme, die in dem erfindungsgemäßen Verfahren verwendet werden, lassen sich somit vorzugsweise aus Ausgangsstämmen von prokaryontischen Organismen herstellen, die rekombinanten Verfahren zugänglich sind, die durch Fermentation kultivierbar sind und die in der Lage sind, SAM in das Kulturmedium zu sekretieren. Bevorzugt handelt es sich um Bakterienstämme aus der Familie Enterobacteriaceae, besonders bevorzugt der Gattung Escherichia, insbesondere bevorzugt um Stämme der Art *Escherichia coli.* Dabei ist insbesondere die Verwendung eines E. coli-Stammes, der keine Fremdgene enthält, bevorzugt.

Eine im Vergleich zu Ausgangsstämmen verstärkte Aktivität der SAM-Synthetase kann grundsätzlich durch verschiedene Ansätze erreicht werden.

Entweder kann das Gen für die SAM-Synthetase in der Art verändert werden, dass das davon codierte Enzym eine höhere Aktivität aufweist als das Ausgangsenzym. Dies kann beispielsweise durch unspezifische oder durch gezielte Mutagenese des Gens für eine SAM-Synthetase erfolgen. Unspezifische Mutationen können zum Beispiel durch chemische Agentien (z.B. 1-Methyl-3-nitro-1-nitrosoguanidin, Ethylmethansulfonsäure u.ä.) und/oder durch physikalische Methoden und/oder durch unter bestimmten Bedingungen durchgeführte PCR-Reaktionen und/oder durch Amplifikation der DNS in Mutatorstämmen (z.B. XL1-Red, Stratagene, Amsterdam, NL) erzeugt werden. Methoden zur Einführung von Mutationen an spezifischen Positionen innerhalb eines DNS-Fragmentes sind bekannt. Eine weitere Möglichkeit zur Erzeugung von SAM-Synthetasen mit im Vergleich zum Ausgangs-Enzym gesteigerter Aktivität besteht in der Kombination verschiedener oben genannter Methoden.

Eine weitere Möglichkeit, eine im Vergleich zu Ausgangsstämmen verstärkte Aktivität der SAM-Synthetase zu erzielen, ist die Überexpression des für dieses Enzym codierenden Gens. Unter einer Überexpression ist im Sinne der vorliegenden Erfindung vorzugsweise zu verstehen, dass das SAM-Synthetase-Gen im Vergleich zum jeweiligen Ausgangsstamm, aus dem das SAM-Synthetase-Gen gewonnen wurde, mindestens um den Faktor 2, bevorzugt mindestens um den Faktor 5, vermehrt exprimiert wird.

Um eine Überexpression des *metK*-Gens in dem Stamm zu erreichen, kann die Kopienzahl des *metK*-Gens in einem Bakterienstamm erhöht sein und/oder es kann die Expression des *metK*-Gens, vorzugsweise durch geeignete Promotoren, gesteigert sein.

Die Erhöhung der Kopienzahl eines *metK*-Gens kann in einer Ausgangsstamm-Zelle mit dem Fachmann bekannten Methoden erreicht werden. So kann zum Beispiel ein *metK*-Gen in einen Plasmid-Vektor mit mehrfacher Kopienzahl pro Zelle (z.B. pUC19, pBR322, pACYC184 für *Escherichia coli)* kloniert und in den Stamm eingebracht werden. Alternativ kann ein *metK*-Gen mehrfach ins Chromosom einer Zelle integriert werden. Als Integrationsverfahren können die bekannten Systeme mit temperenten Bakteriophagen, integrative Plasmide oder die Integration über homologe Rekombination genutzt werden.

Bevorzugt ist die Erhöhung der Kopienzahl durch Klonierung eines *metK*-Gens in einen Plasmid-Vektor unter Kontrolle eines Promotors. Besonders bevorzugt ist die Erhöhung der Kopienzahl in *Escherichia coli* durch Klonierung eines *metK*-Gens in ein pBR322-Derivat wie z. B. pJF118ut (abgeleitet von pJF118EH, Fürste et al. Gene, 119-131 (1986)).

Als Kontrollregion für die Expression eines plasmid-codierten *metK*-Gens kann die natürliche Promotor- und Operatorregion des *metK*-Gens dienen, die verstärkte Expression eines *metK*-Gens kann jedoch insbesondere auch mittels anderer Promotoren erfolgen. Entsprechende Promotorsysteme, die entweder eine andauernde oder eine kontrollierte, induzierbare Expression des SAM-Synthetase-Gens ermöglichen, wie beispielsweise in *Escherichia coli* der konstitutive GAPDH-Promotor des *gapA*-Gens oder die induzierbaren lac-, tac-, trc-, lambda-, ara- oder tet-Promotoren, sind dem Fachmann bekannt. Solche Konstrukte können in an sich bekannter Weise auf Plasmiden oder chromosomal verwendet werden.

In einer besonders bevorzugten Ausführungsform für die Klonierung eines *metK*-Gens wird ein Plasmid verwendet, das bereits einen Promotor zur verstärkten Expression enthält, wie beispielsweise das induzierbare tac-Promotorsystem von *Escherichia coli.*

Des weiteren kann eine verstärkte Expression dadurch erreicht werden, daß Translationsstartsignale, wie z.B. die Ribosomenbindestelle oder das Startcodon des Gens, in optimierter Sequenz auf dem jeweiligen Konstrukt vorhanden sind, oder dass gemäß der "codon usage" seltene Codons gegen häufiger vorkommende Codons ausgetauscht werden, oder dass mRNA-Stabilisierungssequenzen optimiert werden.

Bevorzugt enthalten Bakterienstämme, die im erfindungsgemäßen Verfahren verwendet werden, ein Plasmid mit einem *metK*-Gen sowie den genannten Modifikationen der Regulationssignale.

Die Klonierung eines metK-Gens in einen Plasmid-Vektor erfolgt beispielsweise durch spezifische Amplifikation eines *metK*-Gens mittels der Polymerase-Ketten-Reaktion unter Einsatz von spezifischen Primern, die das komplette *metK*-Gen erfassen, und anschließende Ligation mit Vektor-DNS-Fragmenten.

Die Effektivität eines Bakterienstammes zur erfindungsgemäßen fermentativen Produktion von SAM kann durch zusätzliche Maßnahmen verstärkt werden. Anstelle des Zusatzes von L-Methionin oder D,L-Methionin kann die endogene Methionin-Synthese des im erfindungsgemäßen Verfahren verwendeten Stammes gestärkt werden. Beispielsweise können zu diesem Zweck Stämme verwendet werden, in welchen das Gen *metJ,* welches für einen Repressor der Gene des Methionin- und SAM-Stoffwechsels codiert, nicht mehr exprimiert wird (JP2000139471A) oder Stämme, die eine verbesserte Methionin-Synthese zeigen, weil sie eine verbesserte Homoserin-Transsuccinylase besitzen (JP2000139471A, DE-A-10247437, DE-A-10249642).

Durch eine gängige Transformationsmethode (z.B. Elektroporation, CaCl₂-Methode) werden die *metK*-haltigen Plasmide in einen Ausgangsstamm eingebracht und beispielsweise mittels Antibiotika-Resistenz auf plasmid-tragende Klone selektiert.

Die Kultivierung des Bakterienstammes zur erfindungsgemäßen Produktion von SAM erfolgt vorzugsweise in einem aus der Literatur bekannten Minimalsalzmedium.

Als Kohlenstoffquelle können prinzipiell alle verwertbaren Zucker, Zuckeralkohole, organische Säuren bzw. deren Salze, Stärkehydrolysate, Melasse oder andere Stoffe verwendet werden. Dabei werden bevorzugt Glucose oder Glycerin eingesetzt. Auch ist eine kombinierte Fütterung mehrerer verschiedener Kohlenstoffquellen möglich. Als Stickstoffquellen können Harnstoff, Ammoniak und seine Salze, Nitratsalze sowie andere N-Quellen dienen. Zu den möglichen Stickstoffquellen gehören auch komplexe Aminosäuregemische wie Hefeextrakt, Pepton, Malzextrakt, Sojapepton, Casaminosäuren, Maisquellwasser (Corn Steep Liquor) und NZ-Amine.

Des weiteren können dem Medium bestimmte Komponenten zugegeben werden wie Vitamine, Salze, Hefeextrakt, Aminosäuren und Spurenelemente, durch die das Zellwachstum verbessert wird.

Außerdem kann dem Medium L-Methionin als spezifische Vorstufe für die SAM-Synthese in einer Konzentration zwischen 0,05 und 25 g/l zugesetzt werden. Bevorzugt ist der Zusatz von L-Methionin in der Konzentration zwischen 1 und 5 g/l.

In einem besonders bevorzugten erfindungsgemäßen Verfahren wird dem Medium D,L-Methionin statt L-Methionin in einer Konzentration zwischen 0,05 und 25 g/l zugesetzt. Bevorzugt ist der Zusatz von D,L-Methionin in der Konzentration zwischen 1 und 5 g/l.

Die Inkubation des Stammes erfolgt vorzugsweise unter aeroben Kultivierungsbedingungen über einen Zeitraum von 16 - 150 h und im Bereich der für den jeweiligen Stamm optimalen Wachtumstemperatur.

Als optimaler Temperaturbereich werden 15 - 55 °C bevorzugt. Besonders bevorzugt ist eine Temperatur zwischen 30 und 37 °C.

Die Anzucht des Stammes kann im Schüttelkolben oder Fermenter erfolgen, wobei keine Beschränkungen hinsichtlich Volumen gegeben sind. Die Anzucht kann im Batch-Verfahren, im Fed-Batchoder in einem kontinuierlichen Verfahren erfolgen.

Die Gewinnung von SAM aus dem Kulturmedium kann nach dem Fachmann bekannten Verfahren, wie Zentrifugation des Mediums zur Abtrennung der Zellen und anschließende chromatographische Reinigung, Komplexierung, Filtration wie zum Beispiel Querstromfiltration oder Fällung des Produktes, erfolgen.

Der Nachweis und die Quantifizierung des im erfindungsgemäßen Verfahren produzierten SAM erfolgt beispielsweise mittels Chromatographie (zum Beispiel HPLC).

Die folgenden Beispiele dienen der weiteren Erläuterung der Erfindung. Der Bakterienstamm Escherichia coli W3110/pKP481, der für die Ausführung der Beispiele verwendet wurde, wurde bei der DSMZ (Deutsche Sammlung für Mikroorganismen und Zellkulturen GmbH, D-38142 Braunschweig) unter der Nummer DSM 15426 gemäß Budapester Vertrag hinterlegt.

Sämtliche eingesetzten molekularbiologischen Verfahren, wie Polymerase-Ketten-Reaktion, Isolierung und Reinigung von DNS, Modifikation von DNS durch Restriktionsenzyme, Klenow-Fragment und Ligase, Transformation etc., wurden in der dem Fachmann bekannten, in der Literatur beschriebenen oder von den jeweiligen Herstellern empfohlenen Art und Weise durchgeführt.

### Beispiel 1: Konstruktion des Plasmids pKP481

### A. Amplifizierung des metK-Gens

Das *metK*-Gen aus *E. coli* wurde mittels der Polymerase-Ketten-Reaktion (PCR) unter Verwendung der Taq-DNA-Polymerase nach gängiger, dem Fachmann bekannter Praxis ampliziziert. Als Matrize diente die chromosomale DNA des E. coli-Wildtypstammes W3110 (ATCC 27325). Als Primer wurden die Oligonukleotide metK2 mit der Sequenz 5´-CCTTAATTAATGTCTGTTGTGGTTGGTGT-3´ (SEQ ID No: 3) und metK4 mit der Sequenz 5'-GGAATTCTCTTTAGGAGGTATTAAATATG-3' (SEQ ID No: 4) verwendet.

Das bei der PCR erhaltene DNA-Fragment mit einer Länge von ca. 1,2 kb wurde anschließend mittels eines DNA-Adsorptionssäulchens des QIAprep Spin Miniprep Kits (Qiagen, Hilden) nach Herstellerangaben gereinigt.

### B. Klonierung des metK-Gens in den Vektor pJF118ut

In das PCR-Fragment wurde über Primer metK4 eine Schnittstelle für die Restriktionsendonuklease *EcoRI* eingeführt. Das gereinigte PCR-Fragment wurde mit der Restriktionsendonuclease *Eco*RI unter den vom Hersteller angegebenen Bedingungen geschnitten, anschließend phosphoryliert, über ein Agarosegel aufgetrennt und dann mittels des QIAquick Gel Extraction Kits (Qiagen) nach Herstellerangaben aus dem Agarosegel isoliert. Der aus dem Klonierungs- und Expressionsvektor pJF118EH (Fürste et al. Gene, 119-131 (1986)) abgeleitete Vektor pJF118ut enthält verschiedene genetische Elemente, die eine kontrollierte Expression eines beliebigen Gens erlauben. Es handelt sich dabei um einen Vektor mit einem von der pBR-Plasmidfamilie abgeleiteten Replikationsursprung. Die Expression des klonierten Gens wird durch den tac-Promotor kontrolliert, durch den lacIq-Repressor unterdrückt und kann durch Lactose oder IPTG induziert werden.

Zur Klonierung des *metK*-Gens wurde der Vektor pJF118ut mit den Restriktionsenzymen *Eco*RI und *Pst*I unter den vom Hersteller angegebenen Bedingungen geschnitten. Der 3'-Überhang der PstI-Schnittstelle wurde mittels Klenow-Enzym auf dem Fachmann bekannte Art und Weise abgedaut. Das Plasmid wurde anschließend durch Behandlung mit Alkalischer Phosphatase an den 5'-Enden dephosphoryliert und dann wie das PCR-Fragment mittels QIAquick Gel Extraction Kit (Qiagen) gereinigt. Die Ligation des PCR-Fragments mit dem geschnittenen und dephosphorylierten Vektor erfolgte nach Herstellerangaben unter Verwendung der T4-DNA-Ligase. Die Transformation von *E. coli*-Zellen des Stammes DH5α mit dem Ligationsansatz wurde mittels Elektroporation in einer dem Fachmann bekannten Art und Weise durchgeführt. Der Transformationsansatz wurde auf LB-Ampicillin-Agarplatten (10 g/l Trypton, 5 g/l Hefeextrakt, 5 g/l NaCl, 15 g/l Agar, 20 mg/l Tetracyclin) ausgebracht und über Nacht bei 37 °C inkubiert.

Die gewünschten Transformanten wurden nach einer Plasmidisolierung mittels eines QIAprep Spin Miniprep Kit (Qiagen, Hilden) durch eine Restriktionsanalyse identifiziert und die Fehlerfreiheit durch Sequenzanalyse bestätigt.

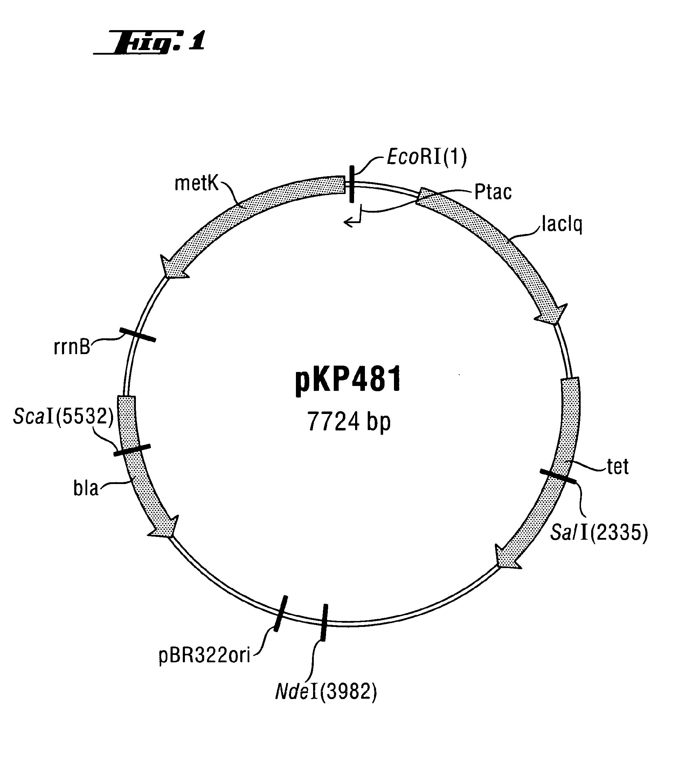

Im dem auf diese Weise erhaltenen Plasmid pKP481 (Fig. 1) ist das *metK*-Gen unter Kontrolle des tac-Promotors.

### Beispiel 2: Herstellung eines Produzenten von S-Adenosylmethionin

Das in Beispiel 1 beschriebene Plasmid pKP481 wurde mittels CaCl₂-Method in den *E. coli*-Stamm W3110 (ATCC 27325) transformiert und nach Selektion auf LB-Agarplatten mit 20 mg/l Tetracyclin wurde das Plasmid aus einer der Transformanten reisoliert, mit Restriktionsendonucleasen gespalten und überprüft. Dieser Stamm wird als W3110/pKP481 bezeichnet und ist für die Produktion von SAM geeignet.

### Beispiel 3: Fermentative Produktion von S-Adenosylmethionin

### A. Produktion von SAM

Für die fermentative Produktion von SAM wurde der Stamm W3110/pKP481 verwendet. Als Vergleich diente der Wildtyp-Stamm W3110 (ATCC 27325) ohne Plasmid, der unter denselben Bedingungen kultiviert wurde.

Für die Anzucht wurde folgendes Medium verwendet: für 1 l Medium: CaCl₂ x 2 H₂O 0,0147 g, MgSO₄ x 7 H₂O 0,3 g, Na₂MoO₄ x 2 H₂O 0,15 mg, H₃BO₃ 2,5 mg, CoCl₂ x 6 H₂O 0,7 mg, CuSO₄ x 5 H₂O 0,25 mg, MnCl₂ x 4 H₂O 1,6 mg, ZnSO₄ x 7 H₂O 0,3 mg, KH₂PO₄ 3,0 g, K₂HPO₄ 12,0 g, (NH₄) ₂SO₄ 5 g, NaCl 0,6 g, FeSO₄ x 7 H₂O 0,002 g, Na₃-Citrat x 2 H₂O 1g, Glucose 15 g, Trypton 1 g, Hefeextrakt 0,5 g. Bei der Anzucht von W3110/pKP481 wurde dem Medium 20 µg/ml Tetracyclin zugesetzt. Wo angegeben (siehe Tabelle) enthielt das Medium außerdem einen Zusatz von 0,5 g/l L-Methionin oder 1 g/l D,L-Methionin.

Als Vorkultur für die Produktionsanzucht wurden zunächst 10 ml Medium in einem 100 ml Erlenmeyerkolben mit dem jeweiligen Stamm beimpft und für 16 h bei 37 °C und 160 rpm auf einem Schüttler inkubiert. Mit den auf diese Weise vorbereiteten Zellen wurden schließlich 50 ml desselben Mediums in einem 300 ml Erlenmeyerkolben auf eine OD₆₀₀ (Absorption bei 600 nm) von 0,1 beimpft. Die Inkubation der Produktionskulturen erfolgte bei 37 °C und 160 rpm auf einem Schüttler für 48 h. Die Expression des SAM-Synthetase-Gens wurde durch Zugabe von 0,1 mM Isopropyl-β-thiogalaktosid (IPTG) bei einer OD₆₀₀ von 0,6 induziert. Nach 24 h und 48 h wurden Proben entnommen und die Zellen durch Zentrifugation vom Kulturmedium abgetrennt.

### B. Quantifizierung des produzierten SAM

Das im Kulturüberstand enthaltene SAM wurde mittels HPLC quantifiziert. Dabei wurde eine Develosil RP-Aqueous C 30- Säule, 5 µm, 250 * 4,6 mm (käuflich erhältlich bei Phenomenex, A-schaffenburg, D) verwendet und 10 µL aufgebrachter Kulturüberstand mittels isokratischer Elution mit einem Fließmittel aus 3 ml 85%iger H₃PO₄ auf 1 l H₂O bei einer Flußrate von 0,5 ml/min bei Raumtemperatur aufgetrennt und mittels Diodenarray-Detektor bei einer Wellenlänge von 260 nm quantifiziert. Tabelle 1 zeigt die erzielten Gehalte von SAM im jeweiligen Kulturüberstand.

**Tabelle 1:**

| **Stamm** | **S-Adenosylmethionin [mg/l]** | | | | | |
|---|---|---|---|---|---|---|
| | **Anzucht ohne Methionin** | | **Anzucht mit 0,5 g/l L-Methionin** | | **Anzucht mit 1 g/l D,L-Methionin** | |
| | **24 h** | **48 h** | **24 h** | **48 h** | **24 h** | **48 h** |
| W3110 | 0 | 0 | 0 | 0 | 0 | 0 |
| W3110/pK P481 | 3 | 12 | 61 | 71 | 34 | 31 |

### Beispiel 4: Konstruktion des Plasmids pMSRLSSk

### A. Amplifizierung des RLSS-Gens

Das Gen für die SAM-Synthetase aus Rattenleber (RLSS; Mato et al., Pharmacol. Ther., 265-280 (1997)) wurde mittels der Polymerase-Kettenreaktion (PCR) unter Verwendung der Taq-DNA-Polymerase nach gängiger, dem Fachmann bekannter Praxis amplifiziert. Als Matrize diente cDNA aus der Ratte (Rattus norvegicus). Als Primer wurden die Oligonukleotide RLSS1 mit der Sequenz 5´-CTAGCAGGAGGAATTCACCATGGGACCTGTGGATGGC-3´ (SEQ ID No: 5) und RLSS2 mit der Sequenz 5´-GGGTACCCCGCTAAAACACAAGCTTCTTGGGGACCTCCCA-3´ (SEQ ID No: 6) verwendet. Das bei der PCR erhaltene DNA-Fragment mit einer Länge von ca. 1,2 kb wurde anschließend mittels eines DNA-Adsorptionssäulchens des QIAprep Spin Miniprep Kits (Qiagen, Hilden) nach Herstellerangaben gereinigt und anschließend phosphoryliert.

### B. Klonierung des RLSS-Gens in den Vektor

Als Basisplasmid für die Konstruktion des erfindungsgemäßen Plasmids wurde das von pACYC184 abgeleitete Plasmid pACYC184-LH verwendet, welches unter der Nummer DSM 10172 bei der Deutschen Sammlung für Mikroorganismen und Zellkulturen in Braunschweig hinterlegt ist. In dieses Plasmid wurde die Sequenz des GAPDH-Promotors eingefügt: Der GAPDH-Promotor wurde durch Polymerase-Kettenreaktion nach den dem Fachmann bekannten Regeln amplifiziert, wobei die Oligonukleotide GAPDHfw mit der Sequenz 5'-GTCGACGCGTGAGGCGAGTCAGTCGCGTAATGC-3' (SEQ ID No. 7) und GAPDHrevII mit der Sequenz 5'-GACCTTAATTAAGATCTCATATATTCCACCAGCTATTTGTTAG-3' (SEQ ID No. 8) als Primer und chromosomale DNS aus E. coli Stamm W3110 (ATCC 27325) als Substrat dienten. Das Produkt wurde elektrophoretisch isoliert, mittels eines QIAquick Gel Extraction Kits (Qiagen) gereinigt und mit den Restriktionsenzymen MluI und PacI nach Herstellerangaben behandelt. Hierauf wurde es in den mit den gleichen Enzymen behandelten Vektor pACYC184-LH mit Hilfe von T4-Ligase eingefügt, wodurch das Plasmid pKP228 entstand.

In das Plasmid pKP228 wurde eine synthetische multiple cloning site eingeführt durch folgendes Vorgehen: pKP228 wurde mit dem Enzym BglII geschnitten, die Enden mit Klenow-Enzym nach Herstellerangaben aufgefüllt und mit Alkalischer Phosphatase dephosphoryliert. In den so präparierten Vektor wurde ein synthetisch hergestelltes doppelsträngiges DNS-Fragment mit folgender Sequenz eingefügt: 5'-GAAGATCTAGGAGGCCTAGCATATGTGAATTCCCGGGCTGCAGCTG-3' (SEQ ID No: 9). Das entstandene Plasmid pKP504 enthält eine multiple cloning site hinter dem GAPDH-Promotor.

pKP504 wurde mit PvuII geschnitten, dephosphoryliert und nach Herstellerangaben mit dem phosphorylierten PCR-Produkt, welches das Gen für die SAM-Synthetase aus Rattenleber (Gensequenz siehe SEQ ID No: 10, Proteinsequenz siehe SEQ ID No. 11) enthält, unter Verwendung der T4-DNA-Ligase ligiert. Die Transformation von *E. coli*-Zellen des Stammes DH5α mit dem Ligationsansatz wurde mittels Elektroporation in einer dem Fachmann bekannten Art und Weise durchgeführt. Der Transformationsansatz wurde auf LB-Ampicillin-Agarplatten (10 g/l Trypton, 5 g/l Hefeextrakt, 5 g/l NaCl, 15 g/l Agar, 20 mg/l Tetracyclin) ausgebracht und über Nacht bei 37 °C inkubiert.

Die gewünschten Transformanten wurden nach einer Plasmidisolierung mittels eines QIAprep Spin Miniprep Kit (Qiagen, Hilden) durch eine Restriktionsanalyse identifiziert und die Fehlerfreiheit durch Sequenzanalyse bestätigt.

Im dem auf diese Weise erhaltenen Plasmid pMSRLSSk ist das RLSS-Gen (codiert für die SAM-Synthetase aus Rattenleber) unter Kontrolle des konstitutiven GAPDH-Promotor des *gapA*-Gens aus Escherichia coli.

### Beispiel 5: Herstellung eines zweiten Produzenten von S-Adenosylmethionin

Das in Beispiel 4 beschriebene Plasmid pMSRLSSk wurde mittels CaCl₂-Methode in den *E. coli*-Stamm W3110 (ATCC 27325) transformiert und nach Selektion auf LB-Agarplatten mit 20 mg/l Tetracyclin wurde das Plasmid aus einer der Transformanten reisoliert, mit Restriktionsendonucleasen gespalten und überprüft. Dieser Stamm wird als W3110/pMSRLSSk bezeichnet und ist für die Produktion von SAM geeignet. Der Stamm wurde bei der DSMZ (Deutsche Sammlung für Mikroorganismen und Zellkulturen GmbH, D-38142 Braunschweig) unter der Nummer DSM 16133 gemäß Budapester Vertrag hinterlegt.

### Beispiel 6: Fermentative Produktion von S-Adenosylmethionin

### A. Produktion von SAM

Für die fermentative Produktion von SAM wurde der Stamm W3110/pMSRLSSk verwendet. Als Vergleich diente der Wildtyp-Stamm W3110 (ATCC 27325) ohne Plasmid, der unter denselben Bedingungen kultiviert wurde.

Für die Anzucht wurde folgendes Medium verwendet: für 1 1 Medium: CaCl₂ x 2 H₂O 0,0147 g, MgSO₄ x 7 H₂O 0,3 g, Na₂MoO₄ x 2 H₂O 0,15 mg, H₃BO₃ 2,5 mg, CoCl₂ x 6 H₂O 0,7 mg, CuSO₄ x 5 H₂O 0,25 mg, MnCl₂ x 4 H₂O 1,6 mg, ZnSO₄ x 7 H₂O 0,3 mg, KH₂PO₄ 3,0 g, K₂HPO₄ 12,0 g, (NH₄) ₂SO₄ 5 g, NaCl 0,6 g, FeSO₄ x 7 H₂O 0,002 g, Na₃-Citrat x 2 H₂O 1g, Glucose 15 g, Trypton 1 g, Hefeextrakt 0,5 g. Bei der Anzucht von W3110/pMSRLSSk wurde dem Medium 20 µg/ml Tetracyclin zugesetzt. Wo angegeben (siehe Tabelle 2) enthielt das Medium außerdem einen Zusatz von 0,5 g/l L-Methionin oder 1 g/l D,L-Methionin.

Als Vorkultur für die Produktionsanzucht wurden zunächst 10 ml Medium in einem 100 ml Erlenmeyerkolben mit dem jeweiligen Stamm beimpft und für 16 h bei 37 °C und 160 rpm auf einem Schüttler inkubiert. Mit den auf diese Weise vorbereiteten Zellen wurden schließlich 50 ml desselben Mediums in einem 300 ml Erlenmeyerkolben auf eine OD₆₀₀ (Absorption bei 600 nm) von 0,1 beimpft. Die Inkubation der Produktionskulturen erfolgte bei 37 °C und 160 rpm auf einem Schüttler für 48 h. Nach 24 h und 48 h wurden Proben entnommen und die Zellen durch Zentrifugation vom Kulturmedium abgetrennt.

### B. Quantifizierung des produzierten SAM

Das im Kulturüberstand enthaltene SAM wurde mittels HPLC quantifiziert. Dabei wurde eine Develosil RP-Aqueous C 30- Säule, 5 µm, 250 * 4,6 mm (käuflich erhältlich bei Phenomenex, A-schaffenburg, D) verwendet und 10 µL aufgebrachter Kulturüberstand mittels isokratischer Elution mit einem Fließmittel aus 3 ml 85%iger H₃PO₄ auf 1 l H₂O bei einer Flußrate von 0,5 ml/min bei Raumtemperatur aufgetrennt und mittels Diodenarray-Detektor bei einer Wellenlänge von 260 nm quantifiziert. Tabelle 2 zeigt die erzielten Gehalte von SAM im jeweiligen Kulturüberstand.

**Tabelle 2:**

| **Stamm** | **S-Adenosylmethionin [mg/l]** | | | | | |
|---|---|---|---|---|---|---|
| | **Anzucht ohne Methionin** | | **Anzucht mit 0,5 g/l L-Methionin** | | **Anzucht mit 1 g/l D,L-Methionin** | |
| | **24 h** | **48 h** | **24 h** | **48 h** | **24 h** | **48 h** |
| W3110 | 0 | 0 | 0 | 0 | 0 | 0 |
| W3110/pM SRLSSk | 9 | 46 | 78 | 82 | 63 | 68 |

## Patentansprüche

1. Verfahren zur fermentativen Produktion von S-Adenosylmethionin (SAM), **dadurch gekennzeichnet, dass** ein Bakterienstamm, der aus einem Ausgangsstamm erhältlich ist, und eine im Vergleich zu dem Ausgangsstamm verstärkte Aktivität der SAM-Synthetase aufweist, in einem Kulturmedium kultiviert wird, wobei der Bakterienstamm SAM in das Kulturmedium ausscheidet und das SAM vom Kulturmedium abgetrennt wird.

2. Verfahren nach Anspruch 1, **dadurch gekennzeichnet, dass** als Bakterienstamm ein Stamm aus der Familie Enterobacteriaceae, besonders bevorzugt der Gattung Escherichia, eingesetzt wird.

3. Verfahren nach Anspruch 1 oder 2, **dadurch gekennzeichnet, dass** als SAM-Synthetase ein Protein umfassend die Sequenz SEQ ID NO: 1 oder eine funktionelle Variante mit einer Sequenzähnlichkeit zu SEQ ID NO: 1 größer 40 %, vorzugsweise größer 60 %, besonders bevorzugt größer 80 %, eingesetzt wird.

4. Verfahren gemäß Anspruch 1 bis 3, **dadurch gekennzeichnet, dass** der Bakterienstamm in einem Minimalsalzmedium kultiviert wird.

5. Verfahren gemäß Anspruch 1 bis 3, **dadurch gekennzeichnet, dass** als Kohlenstoffquelle Glucose oder Glycerin eingesetzt wird.

6. Verfahren gemäß Anspruch 1 bis 3, **dadurch gekennzeichnet, dass** als Stickstoffquelle Harnstoff, Ammoniak oder seine Salze oder Nitratsalze eingesetzt wird.

7. Verfahren gemäß Anspruch 1 bis 3, **dadurch gekennzeichnet, dass** die Inkubation des Bakterienstammes unter aeroben Kultivierungsbedingungen über einen Zeitraum von 16 - 150 h und im Bereich der für den jeweiligen Bakterienstamm optimalen Wachstumstemperatur erfolgt.

8. Verfahren gemäß Anspruch 1 bis 3, **dadurch gekennzeichnet, dass** dem Minimalsalzmedium L-Methionin beigesetzt wird, wobei ein Zusatz in einer Konzentration zwischen 0,05 und 25 g/l bevorzugt, und ein Zusatz in einer Konzentration zwischen 1 und 5 g/l besonders bevorzugt ist.

9. Verfahren nach Anspruch 1 bis 7, **dadurch gekennzeichnet, daß** dem Minimalsalzmedium D,L-Methionin beigesetzt wird, wobei ein Zusatz in einer Konzentration zwischen 0,05 und 25 g/l bevorzugt, und ein Zusatz in einer Konzentration zwischen 1 und 5 g/l besonders bevorzugt ist.

10. Verfahren gemäß Anspruch 1 bis 9, **dadurch gekennzeichnet, dass** die Gewinnung von SAM aus dem Kulturmedium mittels einer Zentrifugation des Kulturmediums und einer anschließenden chromatographischen Reinigung, Komplexierung, Filtration wie zum Beispiel einer Querstromfiltration oder Fällung der SAM erfolgt.
